(19) Europäisches Patentamt / European Patent Office / Office européen des brevets

(11) **EP 3 733 169 A1**

(12) **EUROPEAN PATENT APPLICATION**
published in accordance with Art. 153(4) EPC

(43) Date of publication:
**04.11.2020 Bulletin 2020/45**

(51) Int Cl.:
*A61K 31/047* (2006.01)  *A23L 33/10* (2016.01)
*A23L 33/125* (2016.01)  *A61K 36/07* (2006.01)
*A61P 25/28* (2006.01)

(21) Application number: **18897701.1**

(22) Date of filing: **20.12.2018**

(86) International application number:
**PCT/JP2018/047017**

(87) International publication number:
**WO 2019/131444 (04.07.2019 Gazette 2019/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.12.2017 JP 2017248655**

(71) Applicant: **Kao Corporation**
**Chuo-ku,**
**Tokyo 103-8210 (JP)**

(72) Inventors:
• **OKAHARA, Fumiaki**
**Haga-gun, Tochigi 321-3497 (JP)**

• **KOGA, Yoshitaka**
**Haga-gun, Tochigi 321-3497 (JP)**
• **YANO, Michiko**
**Haga-gun, Tochigi 321-3497 (JP)**
• **ISHIDA, Keiko**
**Haga-gun, Tochigi 321-3497 (JP)**
• **MORI, Takuya**
**Haga-gun, Tochigi 321-3497 (JP)**
• **MISAWA, Koichi**
**Haga-gun, Tochigi 321-3497 (JP)**

(74) Representative: **Vossius & Partner**
**Patentanwälte Rechtsanwälte mbB**
**Siebertstrasse 3**
**81675 München (DE)**

(54) **COGNITIVE FUNCTION IMPROVEMENT AGENT**

(57) To provide an agent for improving a cognitive function effective for improving a cognitive function such as memory and learning ability. An agent for improving a cognitive function, comprising mannitol/a squeeze or an extract of *Agaricus bisporus* as an active ingredient.

[Figure 1]

Y-MAZE (SHORT-TERM MEMORY) TEST
(YOUNG MICE)

RATE OF CHANGE IN SPONTANEOUS ALTERNATION BEHAVIORS (%)

☐ NORMAL DIET INTAKE GROUP
■ HIGH FAT DIET INTAKE GROUP (CONTROL)
▨ HIGH FAT DIET + *AGARICUS BISPORUS* INTAKE GROUP

TIME: N.S.
INTERACTION: $P<0.05$

TIME AFTER INTAKE (MONTHS)

*Ave. ±SE (N=12), 2-way ANOVA followed by Dunnett's post hoc test*

*: $P < 0.05$ (VS CONTROL GROUP)

EP 3 733 169 A1

**Description**

Field of the Invention

The present invention relates to an agent for improving a cognitive function.

Background of the Invention

In recent years, the ratio of elderly to the total population has increased, and a decrease in memory and learning ability with aging and an increase of dementia as a disease have become problems. Examples of primary diseases of dementia include neurodegenerative diseases such as Alzheimer's disease, cerebrovascular disorders caused by cerebral infarction, cerebral hemorrhage, etc., brain tumors, head injury, infectious diseases and metabolic diseases. In particular, the number of patients with Alzheimer's disease is significantly increasing with the progress of aging. Alzheimer's disease causes, for example, a sudden decrease in short-term memory retention, a decrease in memory, and personality disorder, and is therefore a social problem from the view point of nursing care.
Alzheimer's disease involves, for example, atrophy or loss of brain tissue, and causes a decrease of a neurotransmitter, acetylcholine. Although the pathogenic mechanism has not been revealed yet, it has been reported that senile plaques and neurofibrillary tangles appear in the cerebral cortex and the hippocampus, and studies are being conducted from both aspects of amyloid β protein present in the center of senile plaques and tau protein which is a component of neurofibrillary tangles.
Conventionally, acetylcholinesterase inhibitors are used treating dementia and Alzheimer's dementia, but do not fundamentally treat dementia, and it is still difficult to say that the inhibitors have sufficient effects.
Meanwhile, mannitol, one of sugar alcohols, is digestion resistant and thus are used as a low energy sweetener. It has been reported that mannitol has pharmacological action such as an ACE inhibitory action (Patent Literature 1).
Furthermore, *Agaricus bisporus* (scientific name) are humus decomposing fungi which decomposes hay of herbaceous plants such as Gramineae plants. *Agaricus bisporus* is widely eaten as a mushroom and is mainly composed of protein, dietary fiber and sugar alcohol (mannitol). It has been reported that an extract of mushrooms has an action of inhibiting the release of leukotriene and thus can be used as an antiallergic drug (Patent Literature 2), and that the extract has an action of promoting fat cell differentiation (Patent Literature 3). Furthermore, it has been reported that an aqueous extract of mushrooms has an action of enhancing nerve growth factor, NGF (Patent Literature 4). Further, it has been reported that when a lyophilized mushroom was taken, an ability of spatial recognition was improved only in the region in which the concentration was very small (Non-patent literature 1).
(Patent Literature 1) JP-A-2005-306775
(Patent Literature 2) JP-A-H11-217336
(Patent Literature 3) JP-A-2009-263344
(Patent Literature 4) JP-B-4410555

(Non-patent Literature 1) Nutr Res. 2015 Dec; 35 (12): 1079-84.

Summary of the Invention

The present invention provides the following:

(1) An agent for improving a cognitive function, comprising mannitol as an active ingredient.
(2) A food for improving a cognitive function, comprising mannitol as an active ingredient.
(3) An agent for improving a cognitive function, comprising a squeeze or an extract of *Agaricus bisporus* as an active ingredient.
(4) A food for improving a cognitive function, comprising a squeeze or an extract of *Agaricus bisporus* as an active ingredient.
(5) Use of mannitol for producing an agent for improving a cognitive function.
(6) Use of mannitol for producing a food for improving a cognitive function.
(7) Mannitol for use in improvement of a cognitive function.
(8) Non-therapeutic use of mannitol for improvement of a cognitive function.
(9) A method for improving a cognitive function, comprising administering mannitol to a subject or having the subject take mannitol.
(10) Use of a squeeze or an extract of *Agaricus bisporus* for producing an agent for improving a cognitive function.

(11) Use of a squeeze or an extract of *Agaricus bisporus* for producing a food for improving a cognitive function.

(12) A squeeze or an extract of *Agaricus bisporus* for use in improvement of a cognitive function.

(13) Non-therapeutic use of a squeeze or an extract of *Agaricus bisporus* for improving a cognitive function.

(14) A method for improving a cognitive function, comprising administering a squeeze or an extract of *Agaricus bisporus* to a subject or having the subject take the squeeze or the extract of *Agaricus bisporus.*

Brief Description of the Drawings

[Figure 1] Figure 1 is a graph showing an improvement in short-term memory by intake of a squeeze of *Agaricus bisporus* by high fat diet-dependent young mice with decreased memory.

[Figure 2] Figure 2 is a graph showing an improvement in long-term memory by intake of a squeeze of *Agaricus bisporus* in high fat diet-dependent young mice with decreased memory.

[Figure 3] Figure 3 is a graph showing an improvement in short-term memory by intake of a squeeze of *Agaricus bisporus* in high fat diet-dependent aged mice with decreased memory.

[Figure 4] Figure 4 is a graph showing an improvement in long-term memory by intake of a squeeze of *Agaricus bisporus* in high fat diet-dependent aged mice with decreased memory.

[Figure 5] Figure 5 is a graph showing an improvement in memory of senescence-accelerated mice after intake of mannitol.

[Figure 6] Figure 6 is a graph showing an action of enhancing a cognitive function of healthy men and women after a single intake of mannitol.

Detailed Description of the Invention

The present invention relates to provision of an agent for improving a cognitive function effective for improving cognitive function such as memory and learning ability.

The present inventors found that a continuous intake of mannitol or a squeeze of *Agaricus bisporus* containing a large amount of mannitol in an animal with decreased memory and learning ability due to a high fat diet and a senescence-accelerated model animal improves the decreased short-term memory and long-term memory and learning ability.

The agent for improving a cognitive function according to the present invention can effectively improve impairments with a decrease in cognitive function such as memory and learning ability of the brain. The agent can effectively improve impairments with a decrease in short-term memory and long-term memory, in particular, caused by unhealthy lifestyles such as a continuous intake of high fat food, an excessive intake of alcohol, and smoking.

In the present invention, mannitol refers to sugar alcohol of mannose, and is also called mannite. Mannitol in the present invention may be any of D-mannitol or L-mannitol, and D-mannitol is preferred. Mannitol can be obtained by, for example, isolation and purification from a plant containing mannitol by a known method. D-mannitols isolated and purified are commercially available from, for example, B Food Science Co., Ltd. and Mitsubishi Shoji Foodtech Co., Ltd. These commercially available products may also be used in the present invention.

Furthermore, not only isolated mannitol but also an extract, a fraction or a purified product of a plant containing mannitol may be used as mannitol in the present invention.

Examples of plants containing mannitol include seaweeds such as kelp, and mushrooms, and a typical example is *Agaricus bisporus* described later.

It is known that *Agaricus bisporus* contains a large amount of mannitol, and in particular, its fruiting body contains from 20 to 50 g of mannitol per 100 g in terms of dry weight thereof. Thus, a squeeze or an extract of *Agaricus bisporus* may be an alternative to mannitol.

In the present invention, *"Agaricus bisporus"* refers to *Agaricus bisporus* belonging to the genus Agaricus of the family Agaricaceae, and is also called "mushroom" in Japan.

Known varieties of *Agaricus bisporus* include the white variety, off-white variety, cream variety, brown variety and *Agaricus bitorquis* variety. In the present invention, any one of them may be preferably used without limitation.

In the present invention, a part of *Agaricus bisporus* to be used is not particularly limited, and any of the cap, the stem, the fruiting body, the mycelium and the sclerotium may be used. It is preferable to use the fruiting body.

Examples of squeezes of *Agaricus bisporus* include a squeezed juice obtained by squeezing *Agaricus bisporus.* The method for producing squeeze is not particularly limited. For example, the squeeze may be produced by roughly cutting *Agaricus bisporus* and squeezing the cut *Agaricus bisporus* by using a squeezer such as a slow juicer.

Examples of extracts of *Agaricus bisporus* include an extract obtained by extracting from a fruiting body of *Agaricus bisporus* itself, a crushed product thereof, a pulverized product thereof or the squeeze described above.

Any of a polar solvent and a non-polar solvent may be used as a solvent for extraction. Specific examples of the solvents include water; monohydric, dihydric or polyhydric alcohols; ketones such as acetone and methyl ethyl ketone; esters such as methyl acetate and ethyl acetate; linear or cyclic ethers such as diethyl ether and tetrahydrofuran; polyethers such as polyethylene glycol; saturated or unsaturated hydrocarbons such as hexane; aromatic hydrocarbons such as benzene and toluene; halogenated hydrocarbons such as dichloromethane, chloroform, dichloroethane and carbon tetrachloride; pyridines; dimethyl sulfoxide; acetonitrile; carbon dioxide, supercritical carbon dioxide; oil and fat, wax, other oils; and a mixture thereof. Preferred examples thereof include water, alcohol and an aqueous solution thereof. Examples of alcohols include methanol, ethanol, 1,3-butylene glycol, n-propanol, isopropanol, n-butanol, isobutanol, sec-butanol and t-butanol, and ethanol is preferred.

The concentration of alcohol in the above aqueous solution of alcohol (% by volume at 25°C) is preferably 30% by volume or more, more preferably 50% by volume or more, and further preferably 75% by volume or more, and preferably 99.5% by volume or less, more preferably 99% by volume or less, and further preferably 98% by volume or less. The concentration of alcohol in the above aqueous solution of alcohol is preferably from 30 to 99.5% by volume, more preferably from 50 to 99% by volume, and further preferably from 75 to 98% by volume. The aqueous solution of alcohol is preferably a from 30 to 99.5% by volume aqueous solution of ethanol, more preferably a 50 to 99% by volume aqueous solution of ethanol, and further preferably a 65 to 98% by volume aqueous solution of ethanol.

It is preferable that the amount of the solvent to be used for extraction is 1 to 100 mL per 1 g of *Agaricus bisporus* (in terms of dry mass). Conditions of extraction are not particularly limited as long as sufficient extraction can be performed. For example, the extraction time is preferably 1 hour or more, and more preferably 3 hours or more, and preferably 2 months or less, more preferably 5 weeks or less, and further preferably 2 weeks or less. The temperature of extraction is preferably 0°C or more, and more preferably 5°C or more, and preferably is the boiling point of the solvent or less, and more preferably 90°C or less. Usually extraction is performed for a long time at low temperature, whereas for a short time at high temperature.

Means of extraction is not particularly limited and usual means may be used, such as solid-liquid extraction, liquid-liquid extraction, immersion, decoction, exudation, reflux extraction, Soxhlet extraction, ultrasonic extraction, microwave extraction and stirring.

The squeeze or the extract of *Agaricus bisporus* of the present invention may be a roughly purified product as long as the squeeze or the extract conforms to standards acceptable for food or medicine and has the advantageous effects of the present invention. Furthermore, a known technique such as liquid-liquid distribution, solid-liquid distribution, a filtration membrane, activated carbon, an adsorption resin, an ion exchange resin or sedimentation may be used for removal of inert impurities, deodorization or decoloration.

Furthermore, their purity may be increased by combining known separation and purification methods. Examples of purification methods include precipitation with an organic solvent, centrifugation, ultrafiltration membranes, high performance liquid chromatographs and column chromatographs.

Furthermore, the squeeze or the extract of *Agaricus bisporus* of the present invention may be directly used, or may be used in the form of a diluted solution prepared by diluting with an appropriate solvent, or may be formed into a concentrated extract, dry powder or paste. Alternatively, the squeeze or the extract may be lyophilized, and when in use, diluted with a solvent usually used for extraction, such as water, ethanol or a mixture of water and ethanol. The squeeze or the extract may also be included in a vesicle such as liposome, or a microcapsule or the like, and used.

It is preferable that the squeeze or the extract of *Agaricus bisporus* of the present invention contains from 0.1 to 50% by mass, preferably from 0.5 to 45% by mass, and more preferably from 1 to 40% by mass of mannitol.

As shown in Examples described later, mannitol exhibits an action of enhancing a cognitive function when taken by a human; and a squeeze of *Agaricus bisporus* has an action of suppressing and improving a decrease in short-term memory and long-term memory and learning ability of an animal, which is caused by unhealthy lifestyles such as intake of high fat food.

Thus, mannitol or the squeeze or the extract of *Agaricus bisporus* can be an agent for improving a cognitive function, and can also be used for producing the agent.

Mannitol or the squeeze or the extract of *Agaricus bisporus* can also be used for improving a cognitive function. In this regard, the use can be administration to a human or a non-human animal, or can be a use for a specimen derived therefrom. This use may also be a therapeutic use or non-therapeutic use. The term "non-therapeutic" is a concept that does not include medical practice, i.e., a concept not including a method of operation, therapy or diagnosis of a human, more specifically, a concept not including a method of operation, therapy or diagnosis of a human by a doctor or a person instructed by a doctor.

In the present invention, the "cognitive function" means higher function of the brain including judgement, calculation, understanding, learning, thinking, language, spatial recognition, object recognition and memory (short-term memory, long-term memory). The "improvement in cognitive function" means maintenance and improvement in cognitive function, and relief and healing of various symptoms caused by a decrease in cognitive function.

The improvement in cognitive function according to the present invention includes improvement in memory.

As used herein, the "memory" means retaining experience in the past and recollecting it later. The memory includes immediate memory, recent memory, short-term memory and long-term memory. Immediate memory refers to recalling information immediately after newly memorizing the information, which is necessary when, for example, repeating a word. Recent memory is retained longer than immediate memory, and is characterized in that information temporarily disappears from the mind after the information is memorized and until called up. An example thereof is to recollect the name of the person who you met the day before. Short-term memory refers to memory retained for a relatively short time, and having a limited amount of information to be stored at one time. Long-term memory means memory retained over an extended period of time without being forgotten among short-term memories. An example thereof is a memory of one's family in childhood.

Thus, the agent for improving a cognitive function according to the present invention is useful for preventing or treating a disease or a condition showing impairment of cognitive function. Examples of such diseases or conditions showing impairment of cognitive function include dementia (e.g., senile dementia, Alzheimer's dementia, cerebrovascular dementia, posttraumatic dementia, dementia caused by various diseases such as dementia caused by brain tumor, dementia caused by chronic subdural hematoma, dementia caused by normal pressure hydrocephalus, postmeningitic dementia and Parkinson's dementia), non-demented cognitive impairment (e.g., mild cognitive impairment (MCI)), and memory or learning impairment (e.g., memory or learning impairment caused by brain developmental disorder).

The agent for improving a cognitive function according to the present invention may serve as a medicine, a quasi-drug or food for a human or an animal, which has the effect of preventing a decrease in cognitive function or improving or enhancing a cognitive function. The agent may also be a material or a preparation to be mixed in such a medicine, a quasi-drug or food.

The above food includes food, functional food, food for specified health uses, food for patients, and supplements, which have a concept of the effect of preventing a decrease in cognitive function or improving or enhancing a cognitive function, with an indication of the concept as necessary.

When the agent for improving a cognitive function according to the present invention is used as a medicine (including a quasi-drug), the medicine may be administered in any dosage form. Examples of dosage forms include oral administration using, for example, a tablet, a capsule, a granule, a powder or a syrup, and parenteral administration using an injection, a suppository, an inhalant, a percutaneous absorbent or an external agent. A preferred dosage form is oral administration.

These pharmaceutical preparations in various dosage forms may be prepared using mannitol or a squeeze or an extract of *Agaricus bisporus* of the present invention alone, or in appropriate combination with other pharmaceutically acceptable ingredients such as an excipient, a binder, an extender, a disintegrant, a surfactant, a lubricant, a dispersant, a buffering agent, a preservative, a corrective agent, a flavor, a coating agent, a carrier, a diluent.

When the agent for improving a cognitive function according to the present invention is used as a food, examples of forms of food include various food compositions such as bread, cake, noodle, confectionary, jelly, frozen food, ice cream, dairy product and beverage, as well as the same forms as the above preparations for oral administration (e.g., tablet, capsule, syrup).

The food in various forms may be prepared using mannitol or a squeeze or an extract of *Agaricus bisporus* of the present invention alone, or in appropriate combination with other food materials, a solvent, a softening agent, an oil, an emulsifier, an antiseptic, a flavor, a stabilizer, a colorant, an antioxidant, a moisturizing agent, a thickener and the like.

The content of mannitol or the squeeze or extract of *Agaricus bisporus* in the agent for improving a cognitive function according to the present invention (in terms of dry matter of the squeeze or extract) is preferably 0.001% by mass or more, more preferably 0.01% by mass or more, preferably 10% by mass or less, more preferably 5% by mass or less, and preferably from 0.001 to 10% by mass, more preferably from 0.01 to 5% by mass.

The dose or the intake of the agent for improving a cognitive function according to the present invention may vary depending on the condition, body weight, sex, age and other factors of a subject. In the case of oral administration or intake, the dose or the intake of mannitol for an adult (body weight: 60 kg) per day is preferably 1 g or more, more preferably 1.5 g or more, and further preferably 2 g or more, and preferably 5 g or less, more preferably 4 g or less, and further preferably 3 g or less. Furthermore, the dose or the intake is preferably from 1 to 5 g, more preferably from 1.5 to 4 g, and further preferably from 2 to 3 g per day.

The dose or the intake of the squeeze of *Agaricus bisporus* (in terms of dry matter of the squeeze) for an adult per day is preferably 3 g or more, more preferably 5 g or more, and further preferably 7 g or more, and preferably 15 g or less, more preferably 12 g or less, and further preferably 10 g or less. Furthermore, the dose or the intake is preferably from 3 to 15 g, more preferably from 5 to 12 g, and further preferably from 7 to 10 g per day.

The dose or the intake of the extract of *Agaricus bisporus* (in terms of dry matter of the extract) for an adult per day is preferably 3 g or more, more preferably 4 g or more, and further preferably 5 g or more, and preferably 15 g or less, more preferably 12 g or less, and further preferably 10 g or less. Furthermore, the dose or the intake is preferably from 3 to 15 g, more preferably from 4 to 12 g, and further preferably from 5 to 10 g per day.

It is preferable that the agent for improving a cognitive function according to the present invention is administered or taken at the time of meal or feeding, or before a meal or feeding. It is particularly preferable that the agent is administered or taken within 5 minutes to 30 minutes before a meal or feeding.

It is preferable that the subject to which the agent for improving a cognitive function according to the present invention is given is a human with a decreased memory or learning ability or a human who wants to maintain or improve a cognitive function.

For the embodiments described above, the present invention also discloses the following modes.

<1> An agent for improving a cognitive function, comprising mannitol as an active ingredient.

<2> A food for improving a cognitive function, comprising mannitol as an active ingredient.

<3> Use of mannitol for producing an agent for improving a cognitive function.

<4> Use of mannitol for producing a food for improving a cognitive function.

<5> Mannitol for use in improvement of a cognitive function.

<6> Non-therapeutic use of mannitol for improving a cognitive function.

<7> A method for improving a cognitive function, comprising administering mannitol to a subject or having the subject take mannitol.

<8> In the agent or food according to <1> to <4>, the content of mannitol in a preparation thereof is preferably 0.001% by mass or more, more preferably 0.01% by mass or more, preferably 10% by mass or less, more preferably 5% by mass or less, and preferably from 0.001 to 10% by mass, more preferably from 0.01 to 5% by mass.

<9> In <1> to <7>, the dose or the intake of mannitol for an adult (body weight: 60 kg) per day is preferably 1 g or more, more preferably 1.5 g or more, and further preferably 2 g or more, and preferably 5 g or less, more preferably 4 g or less, and further preferably 3 g or less, and the dose or the intake is preferably from 1 to 5 g, more preferably from 1.5 to 4 g, further preferably from 2 to 3 g per day.

<10> An agent for improving a cognitive function, comprising a squeeze or an extract of *Agaricus bisporus* as an active ingredient.

<11> A food for improving a cognitive function, comprising a squeeze or an extract of *Agaricus bisporus* as an active ingredient.

<12> Use of a squeeze or an extract of *Agaricus bisporus* for producing an agent for improving a cognitive function.

<13> Use of a squeeze or an extract of *Agaricus bisporus* for producing a food for improving a cognitive function.

<14> A squeeze or an extract of *Agaricus bisporus* for use in improvement of a cognitive function.

<15> Non-therapeutic use of a squeeze or an extract of *Agaricus bisporus* for improving a cognitive function.

<16> A method for improving a cognitive function, comprising administering a squeeze or an extract of *Agaricus bisporus* to a subject or having the subject take a squeeze or an extract of *Agaricus bisporus.*

<17> In <10> to <16>, the squeeze of *Agaricus bisporus* is a squeeze obtained by squeezing a fruiting body of *Agaricus bisporus.*

<18> In <10> to <16>, the squeeze or the extract of *Agaricus bisporus* comprises preferably from 0.1 to 50% by mass of mannitol.

<19> In the agent or food according to <10> to <13>, the content of the squeeze or the extract of *Agaricus bisporus* in a preparation thereof is preferably 0.001% by mass or more, more preferably 0.01% by mass or more, preferably 10% by mass or less, more preferably 5% by mass or less, and preferably from 0.001 to 10% by mass, more preferably from 0.01 to 5% by mass in terms of dry matter of the squeeze or the extract.

<20> In <10> to <16>, the dose or the intake of the squeeze of *Agaricus bisporus* for an adult (body weight: 60 kg) per day is preferably 3 g or more, more preferably 5 g or more, and further preferably 7 g or more, and preferably 15 g or less, more preferably 12 g or less, and further preferably 10 g or less in terms of dry matter of the squeeze, and the dose or the intake is preferably from 3 to 15 g, more preferably from 5 to 12 g, and further preferably from 7 to 10 g per day.

Examples

Production Example 1 Preparation of squeeze of *Agaricus bisporus*

11.3 kg of a fruiting body of *Agaricus bisporus* was squeezed and extracted with a slow juicer (product name: Hurom Slow Juicer HU-400) in a condition of room temperature. The resulting squeeze was lyophilized to give 308.2 g of a squeeze powder.

The composition of the resulting squeeze of *Agaricus bisporus* was measured in the Food Composition Analysis Center (Table 1).

[Table 1]

| Composition of squeeze of Agaricus bisporus | |
|---|---|
| Squeeze of Agaricus bisporus | g/100 g |
| Carbohydrate* | 41.2 |
| Lipid | 2.1 |
| Protein | 34.4 |
| Dietary fiber | 9.4 |
| Ash | 11.4 |
| Water | 1.5 |
| *Content of mannitol in carbohydrate: 78% | |

Production Example 2 Preparation of extract of Agaricus bisporus

1 L of water was added to 100 g of the squeeze of Agaricus bisporus prepared in Production Example 1, and the mixture was stirred at 100°C for 1 hour to perform hot water extraction. The resulting extract liquid was centrifuged (3,000 rpm, 25°C, 5 minutes, himac CF 7D2 (Hitachi)), and the supernatant was collected and lyophilized. 30.0 g out of 74.7 g of the resulting dry powder was suspended in 300 mL of water. 900 mL of 98% ethanol was added to the suspension and the mixture was allowed to stand for 12 hours at room temperature to perform extraction. The resulting extract liquid was centrifuged (3,000 rpm, 25°C, 5 minutes, himac CF 7D2 (Hitachi)), and the supernatant was collected. Ethanol in the supernatant was distilled off under reduced pressure. The remaining water was lyophilized to give 26.1 g of ethanol extract.

Production Example 3 Preparation of animal feed

The feed composition of the test diet (powdery feed) used in this test is shown in Table 2. The normal diet consisted of 5% corn oil and 66.5% pregelatinized potato starch, and the high fat diet consisted of 30% lipid (25% corn oil + 5% lard) + 13% sucrose. The amounts of ingredients other than those (casein, cellulose, minerals and vitamins) was the same in the normal diet and the high fat diet. In view of the nutritional composition, the squeeze of Agaricus bisporus prepared in Production Example 1 was mixed therein by substituting the ingredients so as to have the same nutritional composition and calories as those of the high fat diet. For the test diets, corn oil, lard, casein, cellulose, AIN 76 mineral mixture, AIN 76 vitamin mixture and pregelatinized potato starch manufactured by Oriental Yeast Co., Ltd. were used, and sucrose fine particles (special grade) manufactured by Wako Pure Chemical Industries, Ltd. was used as sucrose.

[Table 2]

| Feed composition of powdery feed | | |
|---|---|---|
| | Control | Agaricus bisporus |
| Corn oil | 25 | 24.54 |
| Lard | 5 | 4.96 |
| Pregelatinized potato starch | 28.5 | 19.28 |
| Sucrose | 13 | 12.96 |
| Casein | 20 | 13.08 |
| Cellulose | 4 | 3.02 |
| Mineral mixture | 3.5 | 1.18 |
| Vitamin mixture | 1 | 0.96 |
| Squeeze of Agaricus bisporus | 0 | 20 |
| Content in test diet is shown in percentage (w/w) | | |

Example 1 Improvement of memory and learning functions of *Agaricus bisporus* in young mice fed a high-fat diet

(1) Animal and method of breeding

7-week old C57BL/6J male mice (CLEA Japan, Inc.) were transferred (room temperature 23°C, humidity 55 ± 10%, light period 7:00 to 19:00), and fed with food and water ad libitum. At 8 weeks old the mice were acclimated to a powdery feed (CE-2, CLEA Japan, Inc.) for a week, and then grouped so that the body weight and memory and learning function of each group were respectively equivalent to each other. When the mice were 9 weeks old, a high fat diet containing 30% of lipid (high fat diet group) or a 30% high fat diet containing 20% of the squeeze of *Agaricus bisporus* (*Agaricus bisporus* intake group) was fed to the mice as a test diet for 17 weeks (26 weeks old). The mice were fed with food and water ad libitum during the feeding period. Memory and learning function was assessed 2 months after (18 weeks old) and 4 months after (26 weeks old) the start of the test.

(2) Test diet

The powdery feed of the above Production Example 3 having the composition ratio shown in Table 2 was used. The intake was measured 3 times a week during the feeding period.

(3) Y-maze (short-term memory) test

The test was performed before the intake of the test diet and 2 months after the start of the intake to measure short-term memory and learning ability (spatial working memory). Each of the mice was placed at an end of a plastic Y-maze with arms each having a length of 40 cm and a height of 12 cm and forming an angle of 120° (manufactured by Noldus Information Technology). The behavior of the mice was video-recorded for 10 minutes, and the order and the number of entries into the arms were measured. The score of short-term memory and learning ability was assessed based on the rate of change in spontaneous alternation behaviors (see the following equation). Rate of change in spontaneous alternation behaviors (%) = number of spontaneous alternation behaviors / (total number of entries - 2) × 100 (number of spontaneous alternation behaviors: number of entries into the respective arms without overlap)

(4) Novel object recognition (long-term memory) test

The test was performed before the intake of the test diet and 4 months after the start of the intake to measure long-term memory and learning ability (reference memory and object recognition). Each of the mice was placed in a box having a length of 30 cm, a width of 30 cm and a height of 40 cm (manufactured by Noldus Information Technology). The mice were acclimated for 5 minutes per day for 3 consecutive days. Next day, two identical objects (rubber objects wrapped with a blue rubber tape, a 4-cm diameter and 3.5-cm high column) were put in the box (at a position with a space of 10 cm in length and 10 cm in width off the wall). The behavior of the mice was video-recorded for 5 minutes and the number of recognitions (the number of times the mouse approached within 1 cm from the respective objects) was measured (training trial). Next day (24 hours later) each mouse was put in the box in which one of the two objects was substituted with a novel object with a different shape (a glass object wrapped with a red rubber tape, a regular triangular pyramid with a side of 6 cm). The behavior of the mice was video-recorded for 5 minutes and the number of recognitions was measured (retention trial). The score of long-term memory and learning ability was assessed based on the ratio of recognition of novel objects in the retention trial (see the following equation).

$$\text{Ratio of recognition of novel object (\%) = number of recognitions of novel object / total number of recognitions} \times 100$$

(5) Statistical analysis

The results of analysis are shown as the average value (Ave.) ± standard error (SE). For statistical analysis, 2-way ANOVA followed by Dunnett's post hoc test and 1-way ANOVA followed by Dunnett's post hoc test were used. When the P value was 0.05 or less, the difference was determined to be statistically significant.

(6) Results

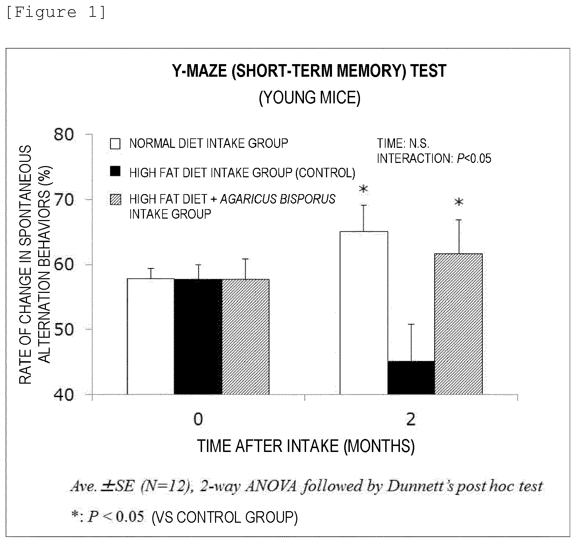

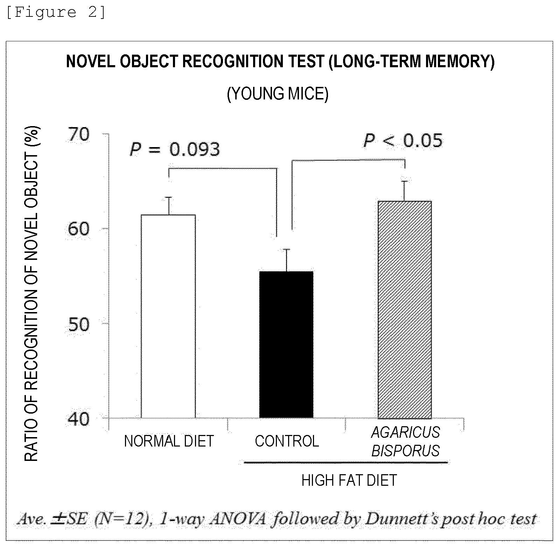

In the Y-maze test, although a significant decrease in the rate of change in spontaneous alternation behaviors (short-term spatial working memory and learning ability) was found in the high fat diet intake group (control) compared with the normal diet intake group, a decrease in short-time memory caused by the continuous intake of the high fat diet was suppressed in the *Agaricus bisporus* intake group (Figure 1). Likewise, in the test of novel object recognition, although a tend to reduce the ratio of recognition of novel objects (long-term object recognition and learning ability) was found in the high fat diet intake group (control) compared with the normal diet intake group, a decrease in long-time memory caused by the continuous intake of the high fat diet was suppressed in the *Agaricus bisporus* intake group (Figure 2).

Example 2 Improvement of memory and learning functions of *Agaricus bisporus* in aged mice fed a high- fat diet

(1) Animal and method of breeding

4-week old C57BL/6J male mice (CLEA Japan, Inc.) were transferred (room temperature 23°C, humidity 55 ± 10%, light period 7:00 to 19:00), and fed and watered ad libitum. The mice were fed with a high fat diet (D12451, Research Diets, Inc) for 57 weeks (until 61 weeks old), and then grouped so that the body weight and memory and learning function of each group was respectively equivalent to each other. A high fat diet containing 30% of lipid (high fat diet group) or a 30% high fat diet containing 20% of the squeeze of *Agaricus bisporus* (*Agaricus bisporus* intake group) was fed to the mice as a test diet for 15 weeks (76 weeks old). The mice were fed with food and water ad libitum during the feeding period. Memory and learning function was assessed 1 month after (65 to 66 weeks old) and 3 months after (75 to 76 weeks old) the start of the test.

(2) Test diet

The powdery feed of the above Production Example 3 having the composition ratio shown in Table 2 was used. The intake was measured 3 times a week during the feeding period.

(3) Y-maze (short-term memory) test

The test was performed before the intake of the test diet and 1 month and 3 months after the start of the intake to measure short-term memory and learning ability (spatial working memory). Each of the mice was placed at an end of a plastic Y-maze with arms each having a length of 40 cm and a height of 12 cm and forming an angle of 120° (manufactured by Noldus Information Technology). The behavior of the mice was video-recorded for 10 minutes, and the order and the number of entries into the arms were measured. The score of short-term memory and learning ability was assessed based on the rate of change in spontaneous alternation behaviors (see the following equation). Rate of change in spontaneous alternation behaviors (%) = number of spontaneous alternation behaviors / (total number of entries - 2) × 100 (number of spontaneous alternation behaviors: number of entries into the respective arms without overlap)

(4) Novel object recognition (long-term memory) test

The test was performed before the intake of the test diet and 1 month and 3 months after the start of the intake to measure long-term memory and learning ability (reference memory and object recognition). Each of the mice was placed in a box having a length of 30 cm, a width of 30 cm and a height of 40 cm (manufactured by Noldus Information Technology). The mice were acclimated for 5 minutes per day for 3 consecutive days. Next day, two identical objects (rubber objects wrapped with a blue rubber tape, a 4-cm diameter and 3.5-cm high column) were put in the box (at a position with a space of 10 cm in length and 10 cm in width off the wall). The behavior of the mice was video-recorded for 5 minutes and the number of recognitions (the number of times the mouse approached within 1 cm from the respective objects) was measured (training trial). Next day (24 hours later) each mouse was put in the box in which one of the two objects was substituted with a novel object with a different shape (a glass object wrapped with a red rubber tape, a regular triangular pyramid with a side of 6 cm). The behavior of the mice was video-recorded for 5 minutes and the number of recognitions was measured (retention trial). The score of long-term memory and learning ability was assessed based on the ratio of recognition of novel objects in the retention trial (see the following equation).

$$\text{Ratio of recognition of novel object (\%) = number of}$$

$$\text{recognitions of novel object / total number of}$$

$$\text{recognitions} \times 100$$

(5) Statistical analysis

The results of analysis are shown as the average value (Ave.) $\pm$ standard error (SE). For statistical analysis, 2-way ANOVA followed by Dunnett's post hoc test was used. When the P value was 0.05 or less, the difference was determined to be statistically significant.

(6) Results

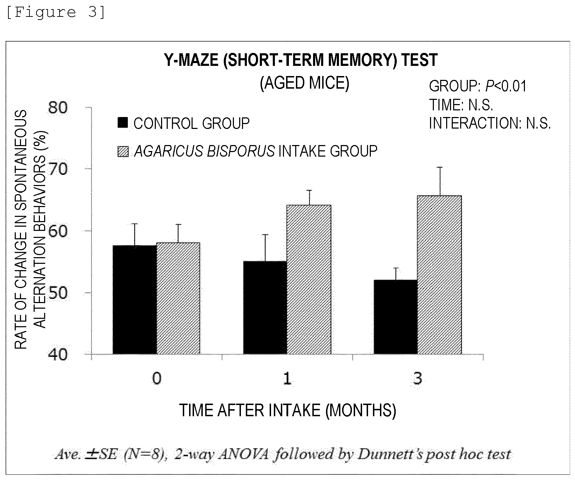

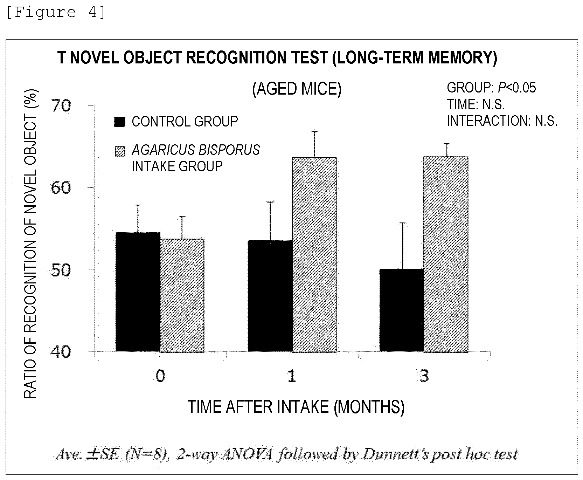

In the Y-maze test, the rate of change in spontaneous alternation behaviors (short-term spatial working memory and learning ability) was increased in the *Agaricus bisporus* intake group compared with the control group (Figure 3). Likewise, in the test of novel object recognition, the ratio of recognition of novel objects (long-time object recognition and learning ability) was increased in the *Agaricus bisporus* intake group compared with the control group (Figure 4).

Example 3 Improvements of memory and learning functions of mannitol in senescence-accelerated mice

(1) Animal and method of breeding

Male senescence-accelerated mice (SAM) P8 (Japan SLC, Inc.), which developed memory and learning impairment associated with age, were transferred at 27 weeks old, and used after a 5-day quarantine period and a 3-day acclimating period. In the acclimating period the body weight was measured and the mice were grouped so that the body weight of each group was equivalent to each other. As a test substances injection water (Otsuka Pharmaceutical Co., Ltd.) or mannitol (50 mg/kg of body weight/day, KAO Corporation) was orally administered once per day for 4 weeks (28 to 32 weeks old) using a stomach tube. After administration of the test substance for 4 weeks (33 weeks old), memory and learning function was assessed. The animals were bred at a room temperature of 23 $\pm$ 3°C and a humidity of 55 $\pm$ 15% with a light period of 6:00 to 18:00. In the test period the animals were fed with usual solid feed (MF, Oriental Yeast Co., Ltd.) and tap water, ad libitum.

(2) Test substance

The method for preparing the test substances used in this test is shown in Table 3. Injection water (Otsuka Pharmaceutical Co., Ltd.) was used as the control solution. A mannitol solution was prepared by adding injection water so that the concentration was 50 mg/mL, stirring and dissolving mannitol. The amount of the solution administered was 10 mL/kg of body weight.

[Table 3]

| Dose of mannitol | | |
| --- | --- | --- |
| | Concentration adjusted | Volume administered |
| Control group | --- | --- |
| Mannitol group | 50mg/mL | 50 mg/kg of body weight/day |

(3) Novel object recognition test

The test was performed 4 weeks after the intake of the test diet to measure memory and learning ability (reference memory and object recognition). Each of the mice was placed in a box having a length of 22 cm, a width of 32 cm and a height of 13 cm (Natsume Seisakusho Co., Ltd.). The mice were acclimated for 10 minutes per day for 2 consecutive days. Next day, two identical objects (building blocks, a regular quadrangular pyramid with a side of 4.5 cm) were put in the box (at a position with a space 2 cm in length and 2 cm in width off the wall). The behavior of the mice was video-recorded for 10 minutes and the time of recognition (the time for which the nose or the forelimb of the mouse touched each object) was measured (training trial). Two hours later each mouse was put in the box in which one of the two objects was substituted with a novel object with a different shape (building blocks, a regular triangular prism with a side of 4.5 cm). The behavior of the mice was video-recorded for 5 minutes and the time of recognition was measured (retention trial). The score of memory and learning ability was assessed based on the ratio of recognition of novel objects in the retention trial (see the following equation).

```
Ratio of recognition of novel object (%) = time of
recognition of novel object / total time of recognition ×
100
```

(4) Statistical analysis

The results of analysis are shown as average value (Ave.) ± standard error (SE). To compare the average value of the control group with that of the mannitol group, unpaired Student's t-test was used. When the P value was 0.05 or less, the difference was determined to be statistically significant.

(5) Results

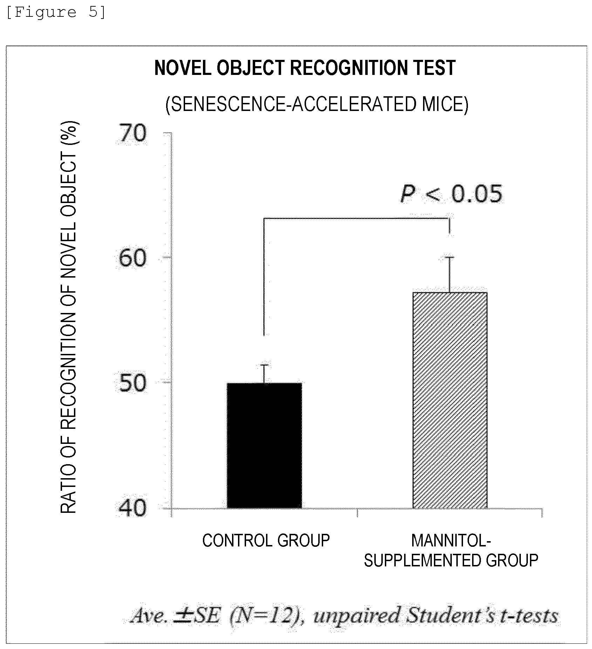

In the test of novel object recognition, an increase in the ratio of recognition of novel objects (object recognition and learning ability) was observed in the mannitol group compared with the control group, indicating that the decrease in memory with aging was suppressed (Figure 5).

Example 4 Cognitive enhancement after single intake of mannitol in healthy men and woman

(1) Overview of Test

Cognitive function of 4 healthy subjects of men and women (average age 38.5 years old ± 9.5, 3 men and a woman) after the intake of a control diet and a diet containing 1.5 g of mannitol powder (D-mannitol, B Food Science Co., Ltd.) (hereinafter described as a mannitol diet) was assessed. The subjects had a specified breakfast at 7:00 a.m., and had the control diet or the mannitol diet as a lunch at 11:30. The following cognitive function tests were performed before the intervention (before lunch, from 10:10) and after the intervention (after lunch, from 12:40). The tests were performed in a crossover design with a washout period of 1 week or more. In two days of the tests in total the same subject had both the control diet and the mannitol diet and measurement was performed.
Control diet: instant cup udon noodle (458 kcal)
Mannitol diet: instant cup udon noodle to which 1.5 g (2.4 kcal) of mannitol powder was added (460.4 kcal)

(2) Assessment of cognitive function

CNS Vital Signs (CNS Vital Signs, LLC, Japanese version) was used for the assessment of cognitive function (Archives of Clinical Neuropsychology, 2006, 21:7:623-643). In the present Examples, cognitive function was assessed based on the following 7 tests.

1) Verbal memory test

First, 15 words are presented, one by one, on a PC screen every 2 seconds, and the subject memorizes them. Immediately or about 30 minutes after that, a larger number of words including 15 new words are presented, and the subject presses the space bar when a word they have memorized appears.

2) Visual memory test

First, 15 geometric figures are presented, one by one, on a PC screen every 2 seconds, and the subject memorizes them. Immediately or about 30 minutes after that, a larger number of figures including 15 new figures are presented, and the subject presses the space bar when a figure they have memorized appears.

3) Finger tapping test

The subject presses the space bar with his/her right index finger as fast as possible for 10 seconds. The test is repeated with the left hand.

4) Symbol Digit Coding (SDC) Test

A table containing combinations of a symbol and a number is presented above on a PC screen. The subject types in the number that corresponds to the symbol in an empty box below on the screen.

5) Stroop test

The stroop test has three parts. In the first part, the words RED, YELLOW, BLUE and GREEN printed in black appear at random on the screen. The subject presses the space bar as soon as the word appears (simple reaction). In the second part, the words RED, YELLOW, BLUE and GREEN appear on the screen, printed in color. The subject presses the space bar when the color of the word matches the meaning of the word (complex reaction). In the third part, the words RED, YELLOW, BLUE and GREEN appear on the screen, printed in color. The subject presses the space bar only when the color of the word does not match the meaning of the word (stroop reaction).

6) Shifting attention test

Three figures (a square or a circle in red or blue) appear on the screen, one on top, two on the bottom. The subject is instructed to follow the rule of matching figures by "shape" or "color" ("match figures by shape" or "match figures by color"), and selects one of two bottom figures, that matches the top figure. The rule of matching instructed ("match figures by shape" or "match figures by color"), and colors (red and blue) and shapes (a circle and a square) of the three figures are changed at random.

7) Continuous performance test

The subject responds to only "B" and press the space bar, out of letters presented at random on the screen, but not to any other letter. The test continues for 5 minutes.

Standardized scores of cognitive function domains were calculated from the results of the tests based on the method of calculation of CNS Vital Signs. The standardized score is calculated by normalization with an average value of 100 and standard deviation of 15 for the subject's age group (in 5-year increments). The results of the tests shown in Table 4 are reflected in the calculation of scores for the respective cognitive function domains.

[Table 4]

| Relation between cognitive domain score and cognitive function test | |
|---|---|
| Cognitive domain | Cognitive function test used for calculation of score |
| Composite memory | Verbal memory, visual memory |
| Verbal memory | Verbal memory |
| Visual memory | Visual memory |
| Psychomotor speed | Finger tapping, symbol digit coding |
| Reaction time | Stroop |
| Complex attention | Stroop, shifting attention, continuous performance |
| Cognitive flexibility | Stroop, shifting attention |
| Processing speed | Symbol digit coding |
| Executive function | Shifting attention |
| Simple attention | Continuous performance |
| Motor speed | Finger tapping |

(continued)

| Relation between cognitive domain score and cognitive function test | |
| --- | --- |
| Cognitive domain | Cognitive function test used for calculation of score |
| Neurocognitive index | Average of the following 5 cognitive domains: composite memory, psychomotor speed, reaction time, complex attention, cognitive flexibility |

(3) Statistical analysis

The values obtained are in the average value $\pm$ standard deviation. To compare the average value of the control diet intake group (control group) with that of the mannitol diet intake group (mannitol-supplemented group), paired t-test was used.

(4) Results

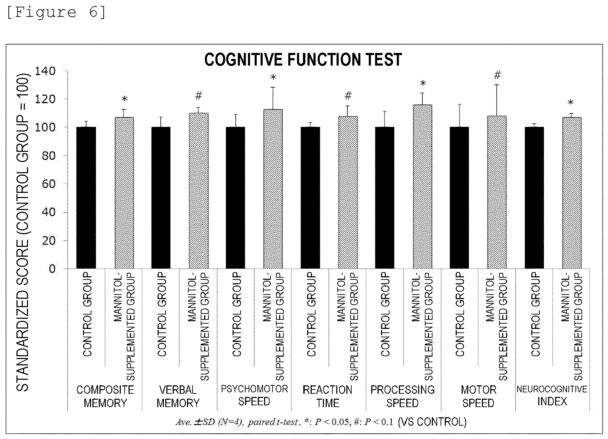

For cognitive domains showing a difference in measurement results after the lunch between the control group and the mannitol-supplemented group, standardized score of the mannitol-supplemented group, the scores are shown as relative values with the standardized score of the control diet intake group as 100 (Figure 6). In the mannitol-supplemented group, the score of composite memory, psychomotor speed, processing speed and neurocognitive index was significantly higher ($P < 0.05$) than those of the control group. The score of verbal memory, reaction time and motor speed also tended to be higher ($P < 0.1$). In the assessment of cognitive function performed before the lunch, there were no cognitive domains showing a difference in standardized scores between the groups. The above results show that a single intake of mannitol enhances cognitive function.

**Claims**

1. An agent for improving a cognitive function, comprising mannitol as an active ingredient.

2. A food for improving a cognitive function, comprising mannitol as an active ingredient.

3. The agent according to claim 1 or the food according to claim 2, wherein the agent or the food is administered or taken in an amount of from 1 to 5 g/60 kg of body weight per day in terms of mannitol.

4. An agent for improving a cognitive function, comprising a squeeze or an extract of *Agaricus bisporus* as an active ingredient.

5. A food for improving a cognitive function, comprising a squeeze or an extract of *Agaricus bisporus* as an active ingredient.

6. The agent according to claim 4 or the food according to claim 5, wherein the squeeze of *Agaricus bisporus* is a squeeze obtained by squeezing a fruiting body of *Agaricus bisporus.*

7. The agent according to claim 4 or 6, or the food according to claim 5 or 6, wherein the squeeze or the extract of Agaricus bisporus comprises from 0.1 to 50% by mass of mannitol.

8. The agent according to claim 4, 6 or 7, or the food according to claim 5, 6 or 7, wherein the squeeze of *Agaricus bisporus* is administered or taken in an amount of from 3 to 15 g/60 kg of body weight per day in terms of dry matter of the squeeze.

9. Use of mannitol for producing an agent for improving a cognitive function.

10. Use of mannitol for producing a food for improving a cognitive function.

11. Mannitol for use in improvement of a cognitive function.

12. Non-therapeutic use of mannitol for improving a cognitive function.

13. A method for improving a cognitive function, comprising administering mannitol to a subject or having the subject take mannitol.

14. The mannitol according to claim 11, wherein mannitol is administered or taken in an amount of from 1 to 5 g/60 kg of body weight per day.

15. The use according to claim 12, wherein mannitol is administered or taken in an amount of from 1 to 5 g/60 kg of body weight per day.

16. The method according to claim 13, wherein mannitol is administered or taken in an amount of from 1 to 5 g/60 kg of body weight per day.

17. Use of a squeeze or an extract of *Agaricus bisporus* for producing an agent for improving a cognitive function.

18. Use of a squeeze or an extract of *Agaricus bisporus* for producing a food for improving a cognitive function.

19. A squeeze or an extract of *Agaricus bisporus* for use in improvement of a cognitive function.

20. Non-therapeutic use of a squeeze or an extract of *Agaricus bisporus* for improving a cognitive function.

21. A method for improving a cognitive function, comprising administering a squeeze or an extract of *Agaricus bisporus* to a subject or having the subject take a squeeze or an extract of *Agaricus bisporus.*

22. The use according to claim 17 or 18, wherein the squeeze or the extract of *Agaricus bisporus* comprises from 0.1 to 50% by mass of mannitol.

23. The squeeze or the extract of *Agaricus bisporus* according to claim 19, wherein the squeeze or the extract comprises from 0.1 to 50% by mass of mannitol.

24. The use according to claim 20, wherein the squeeze or the extract of *Agaricus bisporus* comprises from 0.1 to 50% by mass of mannitol.

25. The method according to claim 21, wherein the squeeze or the extract of *Agaricus bisporus* comprises from 0.1 to 50% by mass of mannitol.

26. The squeeze or the extract of *Agaricus bisporus* according to claim 19, wherein the squeeze of *Agaricus bisporus* is administered or taken in an amount of from 3 to 15 g/60 kg of body weight per day in terms of dry matter of the squeeze.

27. The use according to claim 20, wherein the squeeze of *Agaricus bisporus* is administered or taken in an amount of from 3 to 15 g/60 kg of body weight per day in terms of dry matter of the squeeze.

28. The method according to claim 21, wherein the squeeze of *Agaricus bisporus* is administered or taken in an amount of from 3 to 15 g/60 kg of body weight per day in terms of dry matter of the squeeze.

[Figure 1]

**Y-MAZE (SHORT-TERM MEMORY) TEST**
(YOUNG MICE)

Ave. ±SE (N=12), 2-way ANOVA followed by Dunnett's post hoc test

*: P < 0.05 (VS CONTROL GROUP)

[Figure 2]

NOVEL OBJECT RECOGNITION TEST (LONG-TERM MEMORY)
(YOUNG MICE)

Ave. ±SE (N=12), 1-way ANOVA followed by Dunnett's post hoc test

[Figure 3]

**Y-MAZE (SHORT-TERM MEMORY) TEST**
(AGED MICE)

GROUP: $P<0.01$
TIME: N.S.
INTERACTION: N.S.

■ CONTROL GROUP

▨ *AGARICUS BISPORUS* INTAKE GROUP

RATE OF CHANGE IN SPONTANEOUS ALTERNATION BEHAVIORS (%)

TIME AFTER INTAKE (MONTHS)

*Ave. ±SE (N=8), 2-way ANOVA followed by Dunnett's post hoc test*

[Figure 4]

T NOVEL OBJECT RECOGNITION TEST (LONG-TERM MEMORY)

(AGED MICE)

GROUP: $P<0.05$
TIME: N.S.
INTERACTION: N.S.

CONTROL GROUP

AGARICUS BISPORUS INTAKE GROUP

RATIO OF RECOGNITION OF NOVEL OBJECT (%)

TIME AFTER INTAKE (MONTHS)

Ave. ±SE (N=8), 2-way ANOVA followed by Dunnett's post hoc test

[Figure 5]

**NOVEL OBJECT RECOGNITION TEST**

(SENESCENCE-ACCELERATED MICE)

*P* < 0.05

RATIO OF RECOGNITION OF NOVEL OBJECT (%)

70

60

50

40

CONTROL GROUP

MANNITOL-SUPPLEMENTED GROUP

*Ave. ±SE (N=12), unpaired Student's t-tests*

[Figure 6]

**COGNITIVE FUNCTION TEST**

*Ave. ±SD (N=4), paired t-test, \*: P < 0.05, #: P < 0.1 (VS CONTROL)*

## INTERNATIONAL SEARCH REPORT

| International application No. |
|---|
| PCT/JP2018/047017 |

**A. CLASSIFICATION OF SUBJECT MATTER**

Int.Cl. A61K31/047(2006.01)i, A23L33/10(2016.01)i, A23L33/125(2016.01)i, A61K36/07(2006.01)i, A61P25/28(2006.01)i

According to International Patent Classification (IPC) or to both national classification and IPC

**B. FIELDS SEARCHED**

Minimum documentation searched (classification system followed by classification symbols)
Int.Cl. A61K31/047, A23L33/10, A23L33/125, A61K36/07, A61P25/28

Documentation searched other than minimum documentation to the extent that such documents are included in the fields searched
Published examined utility model applications of Japan 1922–1996
Published unexamined utility model applications of Japan 1971–2019
Registered utility model specifications of Japan 1996–2019
Published registered utility model applications of Japan 1994–2019

Electronic data base consulted during the international search (name of data base and, where practicable, search terms used)
JSTPlus/JMEDPlus/JST7580 (JDreamIII)
CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | THANGTHAENG, N. et al., "Daily supplementation with mushroom (Agaricus bisporus) improves balance and working memory in aged rats", Nutrition Research, 2015, vol. 35, pp. 1079–1084 | 1–10, 12, 13, 15–28<br>11, 14 |
| X<br>A | KHANDELWAL, V. et al., "Comparison of different osmotic therapies in a mouse model of traumatic brain injury", Pharmacological Reports, February 2017, vol. 69, pp. 176–184 | 1, 3, 9, 11, 13–16<br>2, 4–8, 10, 12, 17–28 |
| X<br>A | JP 2013-503853 A (MEDASANI, Munisekhar) 04 February 2013, claims 1, 5, 9, paragraphs [0007], [0024], [0025], example 5 & US 2012/0164245 A1 claims 1, 5, 9, paragraphs [0012], [0034], [0035], embodiment 5 & WO 2011/027363 A2 & EP 2473037 A2 & CN 102497778 A & KR 10-2012-0046795 A | 1–3, 9–16<br>4–8, 17–28 |

☒ Further documents are listed in the continuation of Box C.   ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 21 February 2019 (21.02.2019) | 05 March 2019 (05.03.2019) |

| Name and mailing address of the ISA/<br>Japan Patent Office<br>3-4-3, Kasumigaseki, Chiyoda-ku,<br>Tokyo 100-8915, Japan | Authorized officer<br><br><br>Telephone No. |
|---|---|

Form PCT/ISA/210 (second sheet) (January 2015)

**INTERNATIONAL SEARCH REPORT**

International application No.

PCT/JP2018/047017

C (Continuation).    DOCUMENTS CONSIDERED TO BE RELEVANT

| Category* | Citation of document, with indication, where appropriate, of the relevant passages | Relevant to claim No. |
|---|---|---|
| X<br>A | CN 106722978 A (XUZHOU YITONG FOOD IND CO., LTD.) 31 May 2017, claim 2 (Family: none) | 1-3, 9-16<br>4-8, 17-28 |
| X<br>A | JP 2002-017335 A (YUKIGUNI MAITAKE CO., LTD.) 22 January 2002, claim 1, paragraphs [0007], [0010], [0014], example 2 (Family: none) | 19<br>1-18, 20-28 |
| P, X | WO 2018/124010 A1 (KAO CORP.) 05 July 2018, claims 5, 10, example 4 & JP 2018-104424 A | 1-28 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005306775 A
- JP H11217336 A
- JP 2009263344 A
- JP 4410555 B

**Non-patent literature cited in the description**

- *Nutr Res.,* December 2015, vol. 35 (12), 1079-84